# EUROPEAN PATENT APPLICATION

(11) **EP 1 402 786 A1**
(43) Date of publication of application: **31.03.2004**
(21) Application number: 02256749.9
(22) Date of filing: 27.09.2002
(51) Int. Cl.: A23D 9/00, A23D 7/00, C11B 1/10, C11B 1/02, C11C 1/04, A23L 1/30

(54) **Ximenynic acid**

(71) Applicant: LODERS CROKLAAN B.V., 1521 AX Wormerveer (NL)
(72) Inventor: Koenen, Claudia, NL-1521 AZ Wormerveer (NL); Schmid, Ulrike, NL-1521 AZ Wormerveer (NL); Rogers, Julia, Sharnbrook, Bedford MK44 1LQ (GB); Peilow, Anne, Sharnbrook, Bedford MK44 1LQ (GB); Bosley, John, Kettering, Northants NN14 3JT (GB)
(74) Representative: Stevens, Ian Edward

(57) **Abstract**

Novel blends of compounds comprising Ximenynic acid and glycerides were found that have a number of novel beneficial health effects such as effects on body weight, insuline resistance, Alzheimer disease, cognitive abilities, lipid levels, cancer and skinageing, but also possess structural properties when applied in foods or food suppplements.

## Description

Seed nut oils from trees such as Ximenia Americana (also known as Mountain Plum or Manzanella or Cagalera or Manzana del diabolo or Tallow wood) or Ximenia Africana or Santalum spictum or Santalum acuminatur (also known as Desert quandong or Sandalwood or Katunga or Burn-burn or Mangatais) or Santalum album are known to contain an acetylenic natural fatty acid with the name Ximenynic acid (or octadeca-trans-11-en-9-ynoic acid, 9allt-18:2). The oil is known to have a number of health effects such as :
- activity as leukotriene B4 inhibitor
- activity as phopholipase A2 inhibitor
- activities against peripheral vasculopathies
- activity against headaches
- activity against kidney or heart complaints
- activity against skin ulcers
(cf Biochem Biophys Acta 921 (1987) p.621 - 624 and EP 304603 and information available on Internet).

However the availability and applicability of the acid and derivatives thereof was limited due to the fact that the acid is only present in limited amounts in most of the seed oils of these trees. Moreover the acids so far were only isolated by chromatographic techniques which are not suitable for a commercial application. Therefore there existed a need to make the acid available in a form wherein it could be applied in foods, preferably in a form wherein the acid is dissolved in a system directly applicable in a food. Moreover there was a need to make the acid available in a more concentrated form than available so far and to find new ways to concentrate the acids in a form wherein it could be applied in foods. Further it was desirable to investigate whether the acid and its derivatives had other health benefits, giving broader application.

We studied the above issues and as a result of this study we found novel blends containing Ximenynic acid or a derivative thereof in the form of an alkyl ester with 1 to 11 C-atoms, a glycerol ester (either as mono-di-or triglyceride or as a mix thereof), a wax ester wherein the acid is esterified with an alcohol with 12 or more C-atoms or as a food acceptable salt (in particular Na, K and Ca salts) in amounts that made them suitable for application in foods.

Therefore our invention concerns in the first instance blends comprising Ximenynic acid, or an alkyl or glycerol ester or a wax ester or a food acetable salt thereof, and a glyceride wherein the blends comprise
(i) 0.1 to 99.9 wt %, preferably 1 to 99 wt %, most preferably 2 to 98% of Ximenynic acid, originating from a natural source therefore, or an alkyl, or glycerol ester , or a wax ester or a food acetable salt thereof and
(ii) 0.1 to 99.9 wt %, preferably 1 to 99%, most preferably 2 to 98% of other fatty acids or esters or salts or glycerides, particularly triglycerides, not akin to those present in the natural source of the Ximenynic acid.

The presence of glycerides not akin to the source for the Ximenynic acid makes that the blends can be designed in such a way that they are directly applicable in a food.

Blends that are obtained from Ximenia sources also contain Nervonic acid and in that instance our blends are characterised by the fact that they display a weight ratio of Ximenynic acid to Nervonic acid in the blend of between 0.5 and 5.0, preferably 0.75 - 4.0, most preferably 1.2 to 3.5.We found that for certain applications it is beneficial that Nervonic acid is present as well in the blend.

Concentrated forms of Ximenynic acid have been made by a process which will be discussed later. These concentrates comprise Ximenynic acid or a an alkylester or a glycerol ester or a wax ester or a food acceptable salt thereof in a glyceride and contain at least 15% of Ximenynic acid or the alkylester or glycerol- or wax ester or salt derivative thereof, preferably at least 20% Ximenynic acid and at least 0.5% Nervonic acid or an alkylester or glycerol- or wax ester or a salt thereof, preferably at least 5% Nervonic acid or said derivative thereof.

The other glycerides that can be applied can be selected from a broad group of food grade glycerides, in particular vetable glycerides more particular triglycerides. We prefer to use as the other glycerides, glycerides selected from the group consisting of palm oil; cocoa butter; coconut oil; palm kernel oil; CLA-glycerides; soy bean oil, olive oil; sunflower oil; rape seed oil; safflower oil; corn oil; cotton seed oil; cocoa butter equivalents or cocoa butter replacers; fish oil; borage oil, pine nut oil; coriander oil; fungal oils, or high oleic varieties thereof, or fractions thereof, or hardened varieties thereof, or fractions of the hardened varieties or mixtures of one or more of these oils and fats. It is however also possible to use the free fatty acids corresponding with the hydrolysed fatty acid residues of these glycerides in our blends, in particular in combination with free Ximenynic acid. Also CLA (=Conjugated Linoleic Acid) as free fatty acid can be applied.

The blends suitably are so designed that they display the desired N-values at 5 and 35 °C for the specific application in a food. N-value meaning the solid fat content as measured by NMR-pulse on a mixture that is not stabilised (i.e. the mixture is first melted at 60 to 80 °C, cooled to 0 °C and kept at 0 °C for 30 min whereupon it is heated to measurement temperature kept on this temperature for 30 min and then the N-value is measured). Preferred blends display a solid fat content, measured by NMR pulse on a non stabilised blend at the temperature indicated of N₅ = 5 to 80, preferably 10 to 70 and N₃₅ = less than 20, preferably 1 to 5.

The acids are preferably isolated from Ximenia or from Santalum sp. The glycerides are obtained by extraction from the seeds using a suitable technique, for example solvent extraction or pressing. Esters or ximenynic acid can be produced by esterification with the required alcohol (including glycerol) using enzymic or chemical routes. Food grade salts can be obtained by neutralisation of the acids with the corresponding base.

It was further found that the presence of oxidation stabilisers could prolonge the storage properties of the acids and the blends containing them. In some instances it was even found that the oxidation stabilisers had a synergistic effect on the health properties of the acids. Therefore we prefer to apply blends comprising an effective amount of an oxidation stabiliser preferably selected from the group consisting of natural or synthetic tocopherols, BHT, TBHQ, BHA, propylgallate; free radical scavengers, enzymes with anti oxidant properties and ascorbyl esters of fatty acids. Effective amounts will differ from compound to compound and also will be different for different health effects. However the effective amount can easily be determined by minor experimentation. In general the amount of anti oxidant will vary between 0.05 and 15 wt % (on blend), in particular between 0.1 and 5 wt %.

According to another aspect of our invention it concerns with food products and therefore our invention also comprises food products comprising an effective amount of a health component wherein the health component is Ximenynic acid, or an alkylester or a glycerolester or a wax ester or a food acceptable salt thereof.
Again effective amount can easily be determined by the man skilled in the art without undue experimentation. In general amounts of 0.5 to 10 wt % are very effective.

Food compositions that we prefer are foods selected from the group consisting of margarine; fat continuous or water continuous or bicontinuous spreads, fat reduced spreads; confectionery products such as chocolate or chocolate coatings or chocolate fillings or bakery fillings, ice creams, ice cream coatings, ice cream inclusions, dressings, mayonnaises, cheese, cream alternatives, dry soups, drinks, cereal bars, sauces and snack bars
The application of our novel blends and concentrates is not limited to foods. They also can be applied in food supplements. Therefore part of our invention are also food supplements comprising an effective amount of Ximenynic acid or a derivative thereof in an encapsulating material or in granules or in powder form. In particular supplements, wherein the acid is encapsulated in encapsulating material selected from the group consisting of gelatin, starch, modified starch, flour, modified flour, sugars, in particular sucrose, lactose, glucose and fructose are preferred.

We further found that Ximenynic acid or its derivatives have other novel health effects and these are also part of our invention. In particular we found that Ximenynic acid and its derivatives possessed properties that made them very suitable for use in the preparation of food compositions or food supplements with a health effect, wherein the Ximenynic acid or an alkylester or a glycerolester or a wax ester or a food acceptable salt thereof is used for the preparation of food compositions or food supplements with at least one of the following health effects:
i) lowering or regulation of body weight
ii) prevention or treatment of insulin resistance or related disorders such as diabetes
iii) delaying the onset of symptoms related to development of Alzheimers disease
iv) improving memory function
v) lowering blood lipid levels
vi) skin anti ageing benefits
vii) anti cancer effects

However the acid also displays properties that makes the acid very suitable to be used in compound systems, particularly in food systems to improve / enhance, hardness, texture, aeration, spreadability, oral properties, mouthfeel, flavour impact, colour, viscosity and easiness of processing.

A last embodiment of our invention concerns a new proces for the preparation of an enriched Ximenynic acid concentrate. This method involves a process for the extraction and enrichment of an oil in Ximenynic acid involving the following steps:
(i) Ximenia americana nuts are crushed and boiled with an solvent
(ii) the extract is separated from the solids
(iii) the solvent is removed from the oil
(iv the oil so obtained is partially hydrolysed with a lipase, preferably with Candida rugosa lipase
(v) the partially hydrolysed product is split by physical separation techniques into a fraction enriched in ximenynic acid and in a fraction which is leaner in ximenynic acid.

### EXAMPLES

### EXAMPLE 1

### Extraction of oil from ximenia americana

500g Ximenia americana fruit kernels were thoroughly crushed in a blender. The crushed kernels were put in a 3 liter roundbottom flask and 1,5 liter acetone or hexane was added. The mixture was boiled under reflux for 3 hours. Afterwards, the solids were filtered off and the solvent was removed from the resulting extract by evaporation under vacuum. The resulting oil (180g) was opaque and did not become clear upon heating. The oil was analyzed on its fatty acid composition (see Table I).

**Table I.**

| Fatty acid composition of oil extracted from Ximenia american. kernels with different solvents. | | |
|---|---|---|
| Fatty acid | Extracted in hexane (%) | Extracted in acetone (%) |
| C14:1 | 0.3 | |
| C16:0 | 1.3 | 1.3 |
| C18:0 | 1.1 | 1.2 |
| C18:1 | 41.4 | 41.3 |
| Ximenynic acid | 9.0 | 9.1 |
| C20:0 | 0.3 | 0.3 |
| C20:1 | 1.8 | 1.8 |
| C20:2 | 1.6 | 1.6 |
| C22:0 | 0.8 | 0.8 |
| C22:1 | 1.4 | 1.4 |
| C24:0 | 2.0 | 2.0 |
| C24:1 | 8.4 | 8.4 |
| C26:0 | 1.9 | 1.8 |
| C26:1 | 7.5 | 7.7 |
| C28:0 | 0.7 | 0.7 |
| C28:1 | 11.8 | 11.5 |
| C30:1 | | 2.2 |

### EXAMPLE 2

### Enzymetical enrichment of ximenynic acid in ximenia americana

20g Ximenia americana oil (extracted with acetone) and 7ml demineralized water were put in a 100 ml screwcapped glass bottle. 0.1g Candida *rugosa lipase* in 1ml demineralized water was added and the mixture was homogenized gently. The bottle was put in a shaker (150 rpm) at 35°C. At certain timepoints, samples of the mixture were taken. In these samples, the partially hydrolyzed oil and the FFA were extracted with isooctane and the isooctane was removed by N₂ flow. The percentage FFA in the samples was determined by potentiometric titration. The FFA were absorbed on immobilized tertiary amines and the resulting oil was analyzed on its fatty acid composition by FAME analysis (Table II).

**Table II.**

| Hydrolysis of Ximenia americana oil: Extent of hydrolysis and the percentage of Ximenynic acid in the remaining oil in time. | | |
|---|---|---|
| Time (hours) | Hydrolysis extent (%) | Ximenynic acid in oil (%) |
| 0 | 0.0 | 8.7 |
| 2 | 29.0 | 11.0 |
| 4 | 35.5 | 12.9 |
| 6 | 45.3 | 13.6 |
| 24 | 61.3 | 16.9 |

### EXAMPLE 3

### Effect of the addition of Santalum acuminatum on the activity of acetylcholine esterase

Acetylcholine esterase activity was measured using 96 -well mircoplate reader based on Ellmans method (G.I Ellman, D. Courtney, V. Andres Jr., R.M. Featherstone Biochem. Pharmacol. 7 (1961) 88.).

*Santalum acuminatum* nuts were extracted with hexane to obtain a lipid fraction. 1g of this fraction was hydrolysed to free fatty acids by refluxing with 0.2g of potassium hydroxide in 1.5 mls of ethanol and 0.5 mls of water for 1 hour. The potassium salts were converted to free fatty acids by addition of hydrochloric acid and then extracted into hexane.
The hexane extract of *santalum acuminatum* nuts and the saponified fraction of the hexane extract were dissolved in a mixture of ethanol/ tetrahydofuran/ acetone 60/20/20 at concentrations of 100mg/ml. Serial dilutions were then made as required. These solutions were further diluted 1 to 10 using 50mM Tris-HCL buffer pH 8 *Santalum acuminatum* solutions (20µl), Acetylthiocholine iodide (25µl 15mm in water), 5,5'-dithiobis-(2-nitrobenzoic acid (125µl 3mM in 50mM Tris-HCL buffer pH 8 containing 0.1M NaCl and 0.02M MgCl₂.6H₂O), Tris-HCL buffer (50 µl 50mM pH 8 containing 0.1% bovine serum albumin) were placed in wells together with acetyl choline esterase from electric eel (type VI-s lyophilised powder) (20µl 1.5 µg/ml 50mM Tris-HCL pH 8 buffer containing 0.1% bovine serum albumin)

The absorbance was measured at 410nm every 13s for 8 times. The rate of change of OD of the test materials was compared to the blank solutions.

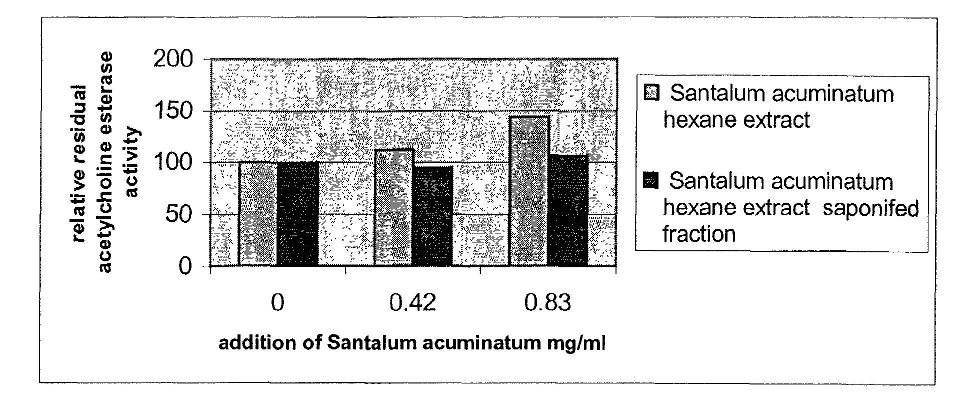

### EXAMPLE 4

### Effect of the addition of ximenia oil saponified fraction on the activity of acetylcholine esterase

Acetylcholine esterase activity was measured using 96 - well mircoplate reader based on Ellmans method (G.I Ellman, D. Courtney, V. Andres Jr., R.M. Featherstone Biochem. Pharmacol. 7 (1961) 88.

Ximenia oil was hydrolysed to free fatty acids by refluxing 1g with 0.2g of potassium hydroxide in 1.5 mls of ethanol and 0.5mls of water for 1 hour. The potassium salts were converted to free fatty acids by addition of hydrochloric acid and then extracted into hexane.
The saponified fraction of ximenia oil was dissolved in a mixture of ethanol/ tetrahydofuran/ acetone 60/20/20 at concentrations of 100mg/ml. Serial dilutions were then made as required. These solutions were further diluted 1 to 10 using 50mM Tris-HCL buffer pH 8
Ximenia solutions (20µl), Acetylthiocholine iodide (25µl 15mm in water), 5,5'-dithiobis-(2-nitrobenzoic acid (125µl 3mM in 50mM Tris-HCL buffer pH 8 containing 0.1M NaCl and 0.02M MgCl₂.6H₂O), Tris-HCL buffer(50 µl 50mM pH 8 containing 0.1% bovine serum albumin) were placed in wells together with acetyl choline esterase from electric eel (type VI-s lyophilised powder) (20µl 1.5µg/ml 50mM Tris-HCL pH 8 buffer containing 0.1% bovine serum albumin)

The absorbance was measured at 410nm every 13s for 8 times. The rate of change of OD of the test materials was compared to the blank solutions.

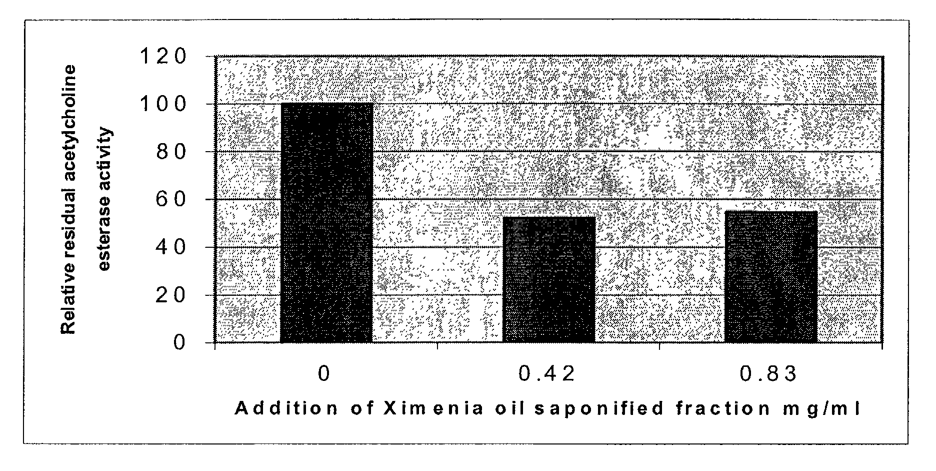

### EXAMPLE 5

### Effect of the addition of Santalum acuminatum fatty acids on the activity of pancreatic lipase

*Santalum acuminatum* nuts were extracted with hexane to obtain a lipid fraction. 1g of this fraction was hydrolysed to free fatty acids by refluxing with 0.2g of potassium hydroxide in 1.5 mls of ethanol and 0.5mls of water for 1 hour. The potassium salts were converted to free fatty acids by addition of hydrochloric acid and then extracted into hexane.

Substrate emulsion (1 ml tributyrin, 3.3 mls emulsification reagent (0.06g sodium chloride, 0.0013g KH₂PO₄, 1.5mls water, 1.8ml glycerol, 0.02g gum arabic), 15.67mls water, 0.027g sodium taurocholate are) were pipetted into an autotitrator vessel together with ximenia oil saponified fraction (added at levels between 0.005 and 0.05g). The mixture was homogenised then the temperature allowed to equilibrate to 30°C and the pH adjusted to approximately 6.5.
Porcine pancreatic lipase solution (0.5 mls of 0.5mg/ml water) was added and the consumption of sodium hydroxide required to maintain a pH of 7 monitored for 6 minutes. The average titration rate was then obtained from the best fit straight line between 2 and 6 minutes.

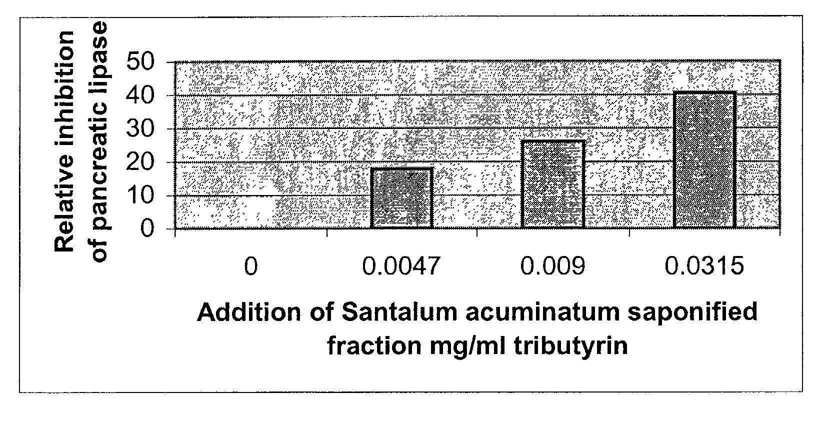

### EXAMPLE 6

### Effect of the addition of ximenia oil saponified fraction on the activity of pancreatic lipase

Ximenia oil was hydrolysed to free fatty acids by refluxing 1g with 0.2g of potassium hydroxide in 1.5 mls of ethanol and 0.5mls of water for 1 hour. The potassium salts were converted to free fatty acids by addition of hydrochloric acid and then extracted into hexane.
Substrate emulsion (1 ml tributyrin, 3.3 mls emulsification reagent (0.06g sodium chloride, 0.0013g KH₂PO₄, 1.5mls water, 1.8ml glycerol, 0.02g gum arabic), 15.67mls water, 0.027g sodium taurocholate are) were pipetted into an autotitrator vessel together with ximenia oil saponified fraction (added at levels between 0.005 and 0.05g). The mixture was homogenised then the temperature allowed to equilibrate to 30°C and the pH adjusted to approximately 6.5.
Porcine pancreatic lipase solution (0.5 mls of 0.5mg/ml water) was added and the consumption of sodium hydroxide required to maintain a pH of 7 monitored for 6 minutes. The average titration rate was then obtained from the best fit straight line between 2 and 6 minutes.

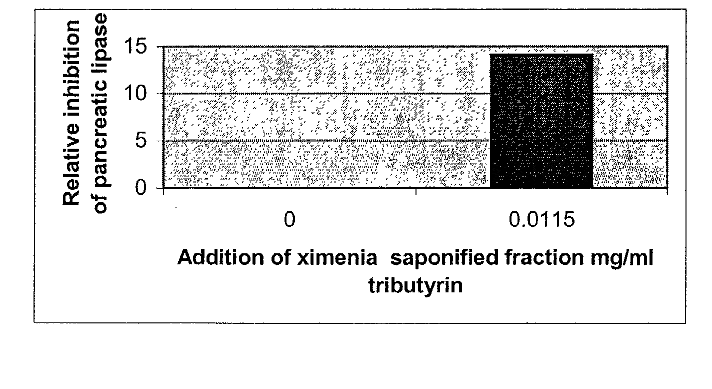

### Example 7

### Effects of ximenia and santalum oil saponifiables (saps) on PPARα (reporter gene assay)

### Cell culture and reporter gene assay

Cos-7 cells (ECACC No. 87021302) were routinely grown in DMEM with 10% FCS (foetal calf serum) at 37°C, 5% CO₂ to 80% confluency. Transient transfections were performed as described by the manufacturers (GibcoBRL). Briefly, cells were plated out in 24 well plates at 50,000 cells per well and incubated overnight in DMEM with 10% FCS at 37°C, 5% CO₂. Cells were then transfected using the LipofectAMINE reagent. For each well, 0.5 µg of DNA mix (pPPRE₃TK-luc 0.40µg; pRL-TK 0.04µg; pcDNA3.1 (-)/PPARα 0.03µg; pRSV/RXRα 0.03µg; in 25µl of DMEM was incubated with 1µl LipofectAMINE, and also in 25µl of DMEM for 45 minutes. The mixture was then made up to 250µl per well and added to the cells, which had been washed with 1ml of DMEM. Cells were then incubated for 5 hours at 37°C, 5% CO₂ and 250µl DMEM with 20% SBCS (charcoal stripped bovine calf serum; Sigma) added. Cells were allowed to recover for 18 hours at 37°C, 5% CO₂ before being treated. The transfection mix was removed from the cells and replaced with treatment mix (DMSO or saponified oil at the specified concentrations) and incubated for 24 hours at 37°C, 5% CO₂. The saponified oils *(Ximenia americana, Santalum acuminatum)* were made up as a stock in DMSO and diluted 1000-fold into DMEM containing 10% SBCS (500µl per well) immediately before being added to the cells. Each treatment was performed in triplicate. Cells were then washed with 1ml of PBS (without calcium or magnesium) and lysed with 100µl per well of 1x Passive Lysis Buffer (as supplied with Promega Dual Luciferase assay kit). Lysis was allowed to continue for 15 minutes and then the lysate was assayed for Firefly and Renilla luciferase activity using the Promega Dual Luciferase assay kit. For the assay 20µl of lysate was taken and assayed as described in the kit instructions using a MLX microtiter plate luminometer (Dynex).
As shown in Fig 1 and 2 both Ximenia and Santalum saps are PPARα ligands. Health effects associated with PPARα activation will include optimising lipid metabolism, prevention and treatment of cancer, anti-inflammatory effects and skin condition and antiageing benefits.

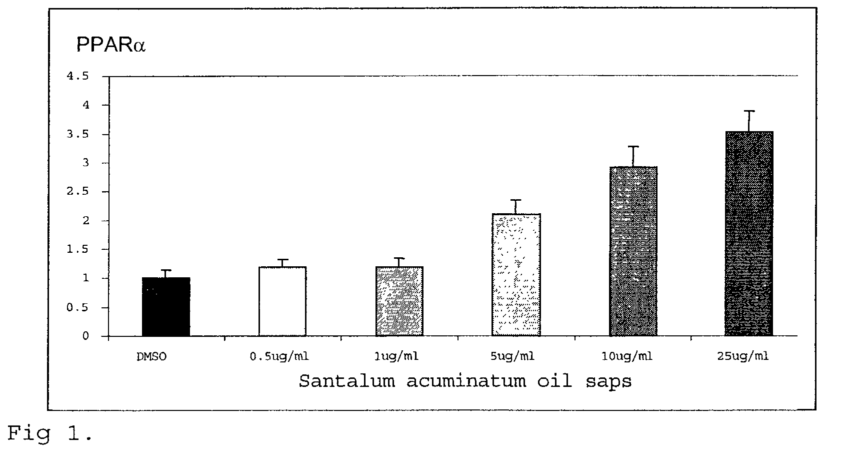

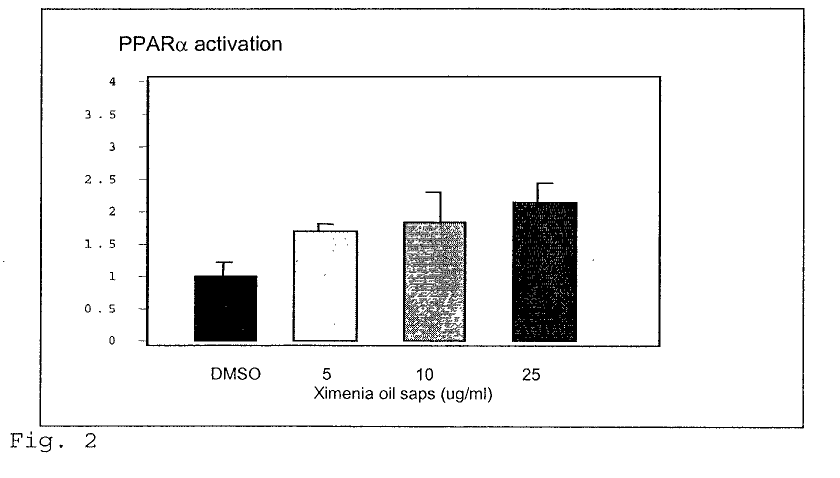

### Example 8

### Effects of santalum oil saponifiables (saps) on PPARγ (reporter gene assay)

### Reporter gene assay

This assay is based on that described by Kliewer et al (Nature 358 771-774 1992). In brief, cos-7 cells (ECACC No. 87021302) were seeded in 24-well plates at a density of 0.325x10⁵ cells/well. Cells were grown overnight at 37°C/5% CO₂ in DMEM containing 10% FCS, 2mM L-glutamine, 100iu/ml penicillin and 100g/ml streptomycin. Cells were washed with transfection media (DMEM containing 2mM L-glutamine) then transiently transfected with 4 plasmids: a PPAR-responsive firefly luciferase reporter gene (pPPRE3TK-luc); mammalian expression plasmids (pcDNA3/hPPAR_{γ}1 and pRSV/hRXRα) containing human PPARγ1 and RXRα cDNAs respectively and a control plasmid (pRLTK, Promega) which constitutatively expresses the renilla luciferase gene. Transfection was performed using Lipofectamine (Gibco Brl) as directed by the manufacturers. Transfected cells were incubated for 6h at 37°C/5% CO₂ and then for a further 46 hours in the presence or absence of ligand (oil saps). After 46 hours cell lysates were prepared and the level of firefly and renilla luciferase determined using the Dual luciferase assay system (Promega) and a MLX microtitre plate luminometer (Dynex). The level of firefly luciferase (normalised against the renilla luciferase control) provides a measure of reporter gene activity. This in turn reflects the level of PPARγ activation.

Fig 3 illustrates that Santalum saps give good activation of PPARγ. This will have benefits in a range of conditions, including in the short term, chronic fatigue, cognitive impairment (memory) and mood swings. Long term benefits would be focused on cardiovascular disease (lipid lowering), type-II diabetes (common in older age) and polycystic ovary syndrome.

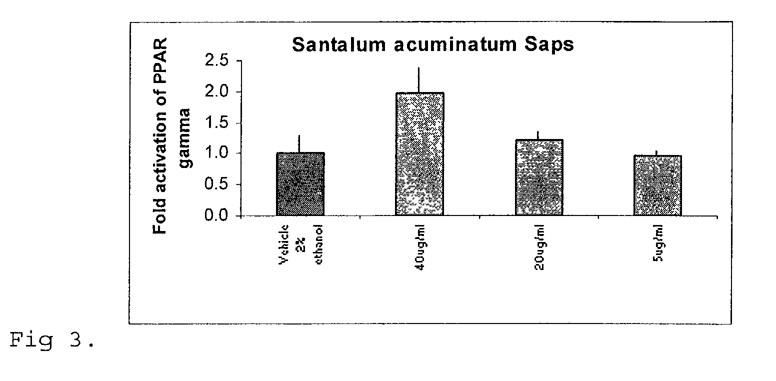

## Claims

1. Blends comprising Ximenynic acid, or an alkyl or glycerol ester or a wax ester or a food acetable salt thereof, and a glyceride wherein the blends comprise
(i) 0.1 to 99.9 wt %, preferably 1 to 99 wt %, most preferably 2 to 98% of Ximenynic acid, originating from a natural source therefore, or an alkyl, or glycerol ester, or a wax ester or a food acetable salt thereof and
(ii) 0.1 to 99.9 wt %, preferably 1 to 99%, most preferably 2 to 98% of other fatty acids or glycerides, particularly triglycerides, not akin to those present in the natural source of the Ximenynic acid.

2. Blends according to claim 1 wherein component (i) of the blend also comprises Nervonic acid and wherein the weight ratio Ximenynic acid to Nervonic acid in the blend is between 0.5 and 5.0, preferably between 0.75 and 4.0, most preferably 1.2 to 3.5.

3. A concentrate of Ximenynic acid or a an alkylester or a glycerol ester or a wax ester or a food acceptable salt thereof in a glyceride having at least 15wt % of Ximenynic acid or the alkylester or glycerol- or wax ester or salt thereof, preferably at least 20% Ximenynic acid and at least 0.5% Nervonic acid or an alkylester or glycerol- or wax ester or a salt thereof, preferably at least 5% Nervonic acid or said derivative thereof.

4. Blends according to claims 1 to 2, containing other glycerides, wherein the other glycerides or derivatives thereof are selected from the group consisting of palm oil; cocoa butter; coconut oil; palm kernel oil; CLA-glycerides ; soy bean oil, olive oil; sunflower oil; rape seed oil; safflower oil; corn oil; cotton seed oil; cocoa butter equivalents or cocoa butter replacers; fish oil; borage oil, pine nut oil; coriander oil; fungal oils, or high oleic varieties thereof, or fractions thereof, or hardened varieties thereof, or fractions of the hardened varieties or mixtures of one or more of these oils and fats, or of the free fatty acids thereof or of free CLA acids

5. Blends according to claim 4 wherein the blends display a solid fat content, measured by NMR pulse on a non stabilised blend at the temperature indicated of N₅ = 5 to 80, preferably 10 to 70 and N₃₅ = less than 20, preferably 1 to 5.

6. Blends according to claims 1 to 5 wherein the Ximenynic acid or derivative thereof is isolated from Ximenia or Santalum species.

7. Blends according to claims 1 to 6 wherein the blends comprise an effective amount of an oxidation stabiliser preferably selected from the group consisting of natural or synthetic tocopherols, BHT, TBHQ, BHA, propylgallate; free radical scavengers, enzymes with anti oxidant properties and ascorbyl esters of fatty acids.

8. Food products comprising an effective amount of a health component wherein the health component is Ximenynic acid, or an alkylester or a glycerolester or a wax ester or a food acceptable salt thereof.

9. Food compositions according to claim 8 wherein the food composition, preferably a fat based composition is selected from the group consisting of margarine; fat continuous or water continuous or bicontinuous spreads, fat reduced spreads; confectionery products such as chocolate or chocolate coatings or chocolate fillings or bakery fillings, ice creams, ice cream coatings, ice cream inclusions, dressings, mayonnaises, cheese, cream alternatives, dry soups, drinks, cereal bars, sauces and snack bars.

10. Food supplements comprising an effective amount of Ximenynic acid or a derivative thereof in an encapsulating material or in granules or in powder form.

11. Food supplements according to claim 10 wherein the encapsulating material is selected from the group consisting of gelatin, starch, modified starch, flour, modified flour, sugars, in particular sucrose, lactose, glucose and fructose.

12. Use of a composition comprising Ximenynic acid, or an alkylester or a glycerolester or a wax ester or a food acceptable salt thereof as active ingredient for the preparation of food compositions or food supplements with a health effect, wherein the Ximenynic acid or an alkylester or a glycerolester or a wax ester or a food acceptable salt thereof is used for the preparation of food compositions or food supplements with at least one of the following health effects:
i) Lowering or the regulation of body weight
ii) prevention or treatment of insulin resistance, or related disorders such as diabetes
iii) delaying the onset of symptoms related to development of Alzheimers disease
iv) improving memory function
v) lowering blood lipid levels
vi) anti cancer effects
vii) Skin antiageing effects

13. Process for the extraction and enrichment of an oil in Ximenynic acid involving the following steps:
(i) Ximenia americana nuts are crushed and boiled with an solvent
(ii) the extract is separated from the solids
(iii) the solvent is removed from the oil
(iv) the oil so obtained is partially hydrolysed with a lipase, preferably with Candida rugosa lipase
(v) the partially hydrolysed product is split by physical separation techniques into a fraction enriched in Ximenynic acid and in a fraction which is leaner in Ximenynic acid.

14. The use of Ximenynic acid or derivatives thereof, in compound systems, particularly in food systems to improve / enhance, hardness, texture, aeration, spreadability, oral properties, mouthfeel, flavour impact, colour, viscosity and easiness of processing.
